(19) Europäisches Patentamt
European Patent Office
Office européen des brevets

(11) **EP 1 764 404 B1**

(12) **EUROPEAN PATENT SPECIFICATION**

(45) Date of publication and mention
of the grant of the patent:
**04.11.2009 Bulletin 2009/45**

(51) Int Cl.:
*C09K 15/30* (2006.01)     *C08F 2/38* (2006.01)
*C07D 279/28* (2006.01)     *C08F 290/06* (2006.01)
*A61K 6/00* (2006.01)

(21) Application number: **05020181.3**

(22) Date of filing: **16.09.2005**

(54) **Use of methylene blue as polymerization inhibitor**

Verwendung von Methylenblau als Polymerisationsinhibitor

Utilisation de bleu de methylène comme inhibiteur de polymérisation

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**21.03.2007 Bulletin 2007/12**

(60) Divisional application:
**09000633.9 / 2 053 066**

(73) Proprietor: **Straumann Holding AG**
**CH-4002 Basel (CH)**

(72) Inventor: **Molenberg, Aaldert**
**4054 Basel (CH)**

(74) Representative: **Schaad, Balass, Menzl & Partner AG**
**Dufourstrasse 101**
**Postfach**
**8034 Zürich (CH)**

(56) References cited:
**EP-A- 0 334 500     DD-A- 215 699**
**GB-A- 942 318     US-A- 4 021 310**

- **DATABASE CA [Online] CHEMICAL ABSTRACTS SERVICE, COLUMBUS, OHIO, US; INSTITUTUL DE CERCETARI PRODUSE AUSILIARE ORGANICE, MEDIAS, ROM.: "Triethylene glycol dimethylacrylate" XP002366213 retrieved from STN Database accession no. 100:176196 & RO 81 797 B 1 June 1983 (1983-06-01)**
- **PATENT ABSTRACTS OF JAPAN vol. 005, no. 152 (C-073), 25 September 1981 (1981-09-25) & JP 56 084708 A (MITSUBISHI GAS CHEM CO INC; others: 01), 10 July 1981 (1981-07-10)**
- **PATENT ABSTRACTS OF JAPAN vol. 1996, no. 10, 31 October 1996 (1996-10-31) & JP 08 155024 A (NIPPON ELECTRIC GLASS CO LTD), 18 June 1996 (1996-06-18)**
- **L. LEVY: "Inhibition of acrylic acid polymerization by phenothiazine and p-methoxyphenol.II. Catalytic inhibition by phenothiazine" J.POLYMER SCIENCE: PART A: POLYMER CHEMISTRY, vol. 30, 1992, pages 569-576, XP009061210**
- **H. VOGEL ET AL: "Aspects of the safe storage of acrylic monomers: Kinetics of the oxygen consumption" CHEM.ENG.TECHNOL., vol. 21, no. 10, 1998, pages 829-837, XP002366196**

**Description**

The present invention relates to the prevention of premature polymerization during the transportation and storage of a polymerizable compound with at least one conjugated unsaturated group by addition of methylene blue as inhibitor.

Polymerizable compounds with at least one conjugated unsaturated group are widely used as basic chemicals. Examples are acrylic monomers, in particular acrylic acid and methacrylic acid. They are mostly used for the synthesis of polymers. In recent times such polymerizable compounds have been used in medicine.

Polymerizable compounds with at least one conjugated unsaturated group are also known to be used for forming biodegradable materials. In WO 01/92584 a matrix material is disclosed which is formed by nucleophilic addition reaction to conjugated unsaturated groups.

WO 00/44808 also discloses a polymeric biomaterial formed by nucleophilic addition reaction to conjugated unsaturated groups. The obtained hydrogels may be used for example as glues or sealants and as scaffolds for tissue engineering and wound healing applications.

During the manufacture, transport and storage of these polymerizable compounds with at least one conjugated unsaturated group, the so-called "runaway" polymerization has to be avoided, since this leads not only to quality loss of the monomer but also to safety problems. Especially when used in medicine such compounds have to fulfil purity requirements, since polymerization would impede stoichiometry of the nucleophilic addition reaction and cause premature gelation of a product. The research of past decades on the inhibition of polymerization of for example acrylic monomers has produced many usable inhibitors to prevent the radical polymerization.

An example a powerful inhibitor is a methyl ether of hydroquinone (MEHQ), which is usually present in acrylic acid at a concentration of approximately 200 ppm (S. Schulze, H. Vogel, Chem. Eng. Technol. 21, 829-836 (1998)). Said inhibitor works only in the presence of oxygen. Therefore MEHQ is not suitable for compositions which are to be used in medicine. Oxygen could impair the longterm stability of components of these compositions, e.g. peroxides could be formed in poly(ethylene glycol), and should therefore not be present in such compositions.

An other inhibitor which is industrially used to inhibit polymerization of said polymerizable compounds is phenothiazine (PTZ). In contrast to MEHQ, PTZ is also a good inhibitor in the absence of oxygen (Levy, J. Polymer Science: Part A: Polymer Chemistry, Vol 30, 569-576 (1992)). Said compound is known for its insecticidal, fungicidal, antibacterial and anthelmintic properties. Due to its widespread use in animals and man also many adverse reactions are known encompassing effects on blood elements, neuromuscular problems and photosensitization (Mitchell, S.C., the toxicity of phenothiazine, Drug Metabolism and Drug Interactions, 11, 204-235 (1994)). Due to said adverse reaction phenothiazine-based inhibitors should be replaced in compositions which are to be used in medicine.

US 4,012,310 discloses the use of an aromatic compound in combination with a copper compound in the presence of molecular oxygen to inhibit polymerization during distillation of acrylic acid or acrylic esters. The described method is not suitable for compositions which are to be used in medicine, not only because oxygen is necessary (cf. above), but also because copper compounds are well known for their toxicity.

GB-A-942 318 discloses an improvement in the process of the interaction of acetone cyanohydrine with sulphuric acid, oleum or chlorosulphonic acid to form methacrylamide and/or a sulphuric acid derivative thereof. The reaction is carried out in the presence of a stabilizer, which is a compund from the phenothiazine, phenoxazine and phenazine series, for instance phenothiazine or methylene blue.

DD 215 699 A discloses a dental composition, which is temperable using light. The dental compound comprises a photo initiator and a sesiblizer dye, for instance methylene blue, to promote polymerization.

Methylene blue is a well known dye. It is used as dye in medicine and is known to be biocompatible.

By "conjugated unsaturated group" the alternation of carbon-carbon, carbon-heteroatom or heteroatom-heteroatom multiple bonds with single bonds is meant.

By "polymerizable compound" is meant that said compound can undergo a polymerization reaction.

The problem of the present invention is to provide an inhibitor to prevent the polymerization of polymerizable compounds with at least one conjugated unsaturated group, wherein the polymerizable compound comprises a core which carries 2 to 10 chains comprising said conjugated unsaturated groups, whereby said conjugated unsaturated groups are attached to any of the last 20 atoms of the chain which may be used in medicine.

The problem is solved by a composition according to claim 1. Further preferred embodiments are subject of claims 2 to 12.

Surprisingly it has been found that methylene blue has the ability to prevent the polymerization of polymerizable compounds with at least one conjugated unsaturated group wherein the polymerizable compound comprises a core which carries 2 to 10 chains comprising said conjugated unsaturated groups, whereby said conjugated unsaturated groups are attached to any of the last 20 atoms of the chain. In the absence of oxygen methylene blue inhibits said polymerization reaction even better than the well known inhibitor phenothiazine. Methylene blue has to be present in a concentration of 10 to 5000 ppm, preferably 20 to 1000 ppm, to inhibit the polymerization of the polymerizable compounds over a long period of time. Surprisingly it could be shown that in absence of oxygen a composition comprising 100 ppm methylene blue is significantly better stabilized than a composition comprising 10 times more phenothiazine (1000 ppm).

Due to the fact that methylene blue has the ability to function as inhibitor in the absence of oxygen, said inhibitor may be used in all fields in which the presence of oxygen is undesired. As mentioned before, for example in pharmaceutical compositions the presence of oxygen should be avoided since oxygen could impair the stability of said compositions. Such compositions are generally in an inert atmosphere such as under argon or under nitrogen. Said compositions are oxygen-free or oxygen-poor, which means that the concentration of oxygen is less than 5%, preferably less than 2%, and most preferably less than 0.5%. It has been found that the absence of oxygen does not influence the activity of methylene blue as inhibitor of the polymerization reaction.

The blue colour of the composition according to the present invention has a positive side effect when used in medicine, since the visibility of the medicinal product is improved.

Polymerizable compounds which are used to form biodegradable matrix material are generally difficult to stabilize since they have to be stored under oxygen-free or oxygen-poor conditions and since the stabilizer has to be biocompatible. Said compounds are especially difficult to stabilize if they have a high number of short chains, and thus a high density of acrylates, resulting in a strong tendency to spontaneously polymerize. It has been found that said compounds which are described below are particularly well stabilized in the presence of methylene blue.

Polymerizable compounds which are used to form biodegradable matrix material comprise a core which carries 2 to 10 chains with a conjugated unsaturated group attached to any of the last 20 atoms of the chain. In a preferred embodiment said conjugated unsaturated group is terminal. The core can be a single atom such as a carbon or a nitrogen atom or small molecules such as an ethylene oxide unit, a sugar, a multifunctional alcohol, such as pentaerythritol or hexaglycerol. The chains are linear polymers or linear or branched alkyl chains optionally comprising heteroatoms, amide groups or ester groups. Beside the chains the core may be additionally substituted with linear or branched alkyl residues or polymers which have no conjugated unsaturated groups. In a preferred embodiment the compound has 4 to 8 chains.

The conjugated unsaturated group is preferably selected from the group consisting of acrylates, acrylamides, quinines, and 2- or 4-vinylpyridiniums.

In a most preferred embodiment the chains of the polymerizable compounds are linear polymers. Said polymers are preferably selected from the group consisting of poly(ethylene glycol), poly(vinyl alcohol), poly(ethylene-co-vinyl alcohol), poly(acrylic acid), poly(ethylene-co-vinyl-pyrrolidone), poly(ethyloxazoline), poly(vinyl pyrrolidone), poly(ethylene-co-vinyl pyrrolidone), poly(maleic acid), poly(ethylene-co-maleic acid), poly(acrylamide) or poly ethylene-co-poly (propylene oxide) block copolymers. Said polymers can also be copolymers, block copolymers, graft copolymers, or random copolymers. Blocks, which are polymerized on the ends of the hydrophilic polymers, can be composed of, for example, lactic acid, glycolic acid, ε-caprolactone, lactic-co-glycolic acid oligomers, trimethylene carbonate, anhydrides, and amino acids.

In a most preferred embodiment the chains of the polymerizable compounds are poly (ethylene glycol) molecules (PEG).

It has been found that methylene blue stabilizes particularly well an eight-arm branched PEG acrylate from hexaglycerol as shown below:

$$\text{Hexaglycerol } [-(CH_2CH_2O)_n-CH_2CH_2-O-CO-CH=CH_2]_8$$

wherein n is 2 to 200, preferably 3 to 10.

To obtain the stabilized composition according to the present invention the following procedure is preferred. Immediately after purification of the compound with at least one conjugated unsaturated group and before evaporation of the solvent, methylene blue is added to said compound. After mixing the components the solvent is evaporated which results in the stabilized composition according to the present invention.

The composition according to the present invention may additionally comprise ethanol which increases the solubility of methylene blue in the compound with at least one conjugated unsaturated group. To obtain such a composition ethanol is added after evaporation of the solvent.

The composition according to the present invention may be used in different fields. Especially preferred are fields in which the absence of oxygen and biocompatibility are important. Preferably the composition according to the present invention is used in medicine, in particular in dentistry.

## Example 1

Samples of 8-arm PEG-acrylate 2k with different inhibitors were weighed into DSC pans and were heated until they had polymerized. Analysis was performed according to the ASTM698 method:

for each sample DSC traces (energy released vs. temperature) were recorded at different heating rates ($\beta$ = 4, 7,

10, and 13 K/min). The temperatures $T_{max}$ at which the polymerization peaks showed a maximum were recorded and the activation energy for the polymerization ($E_a$) was determined from eq. 1:

$$\ln \frac{\beta}{T_{max}^2} = const - \frac{E_a}{RT_{max}} \qquad (1)$$

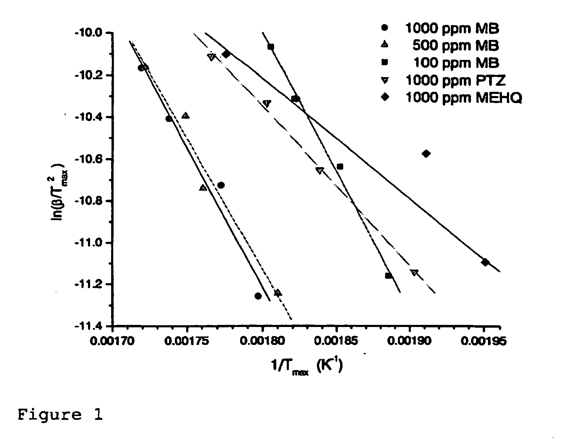

Figure 1 shows the data for 5 different samples.

The preexponential factor A was determined by eq. (2):

$$A = \frac{E_a}{RT_{max}^2} \beta \exp\left(\frac{E_a}{RT_{max}}\right) \qquad (2)$$

The values of A were averaged over the four heating rates. The values of $E_a$ and average values of A are shown in table 1.

<div align="center">

Table 1

| | $E_a$ (kJ/mol) | A (min$^{-1}$) |
|---|---|---|
| 1000 ppm MB | 110 | $4.4 \cdot 10^9$ |
| 500 ppm MB | 110 | $1.3 \cdot 10^{10}$ |
| 100 ppm MB | 104 | $1.0 \cdot 10^9$ |
| 1000 ppm PTZ | 63 | $2.2 \cdot 10^5$ |
| 1000 ppm MEHQ | 48 | $6.4 \cdot 10^3$ |

</div>

By assuming the first order kinetics, ASTM698 permits evaluating the time ($t_\alpha$) to reach a certain extent of reaction ($\alpha$) as follows:

$$t_\alpha = \frac{-\ln(1-\alpha)}{A \exp\left(\frac{-E_a}{RT}\right)} \qquad (3)$$

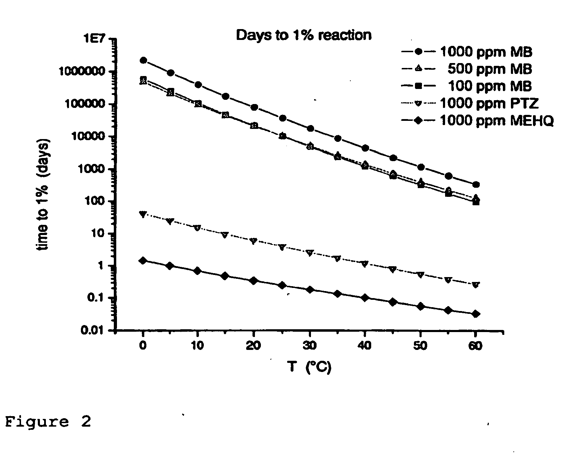

Using the experimentally evaluated values of $E_a$ and A, for each of the samples times to reach 1% of conversion ($\alpha$ = 0.01 in eq. 3) at different temperatures T were estimated. The results are shown in Figure 2.

Figure 2 clearly shows the superior stability of the methylene blue stabilized samples compared to the PTZ and MEHQ stabilized samples.

**Example 2**

In order to establish a relationship between methylene blue (MB) concentration and stability of 8-arm PEG-acrylate 2k, a stress test has been performed.

Samples of one freshly synthesized 8-arm PEG-acrylate 2k batch (Nektar, batch# CM030703) were spiked with different concentrations of MB (250, 500, 1000, and 2000 ppm). For each MB concentration two 3 gram samples were added into separate 20 ml test tubes, which were closed with covers with argon inlets. The tubes were kept at 70°C under argon and the samples were checked daily until they had polymerized, as observed by solidification of the PEG oil.

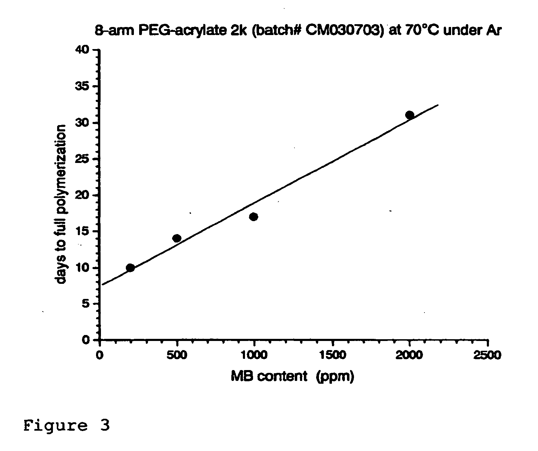

Figure 3 shows the results of the experiment. The linear relationship between MB content and stability confirms that the stabilizing effect is exerted by methylene blue.

### Example 3

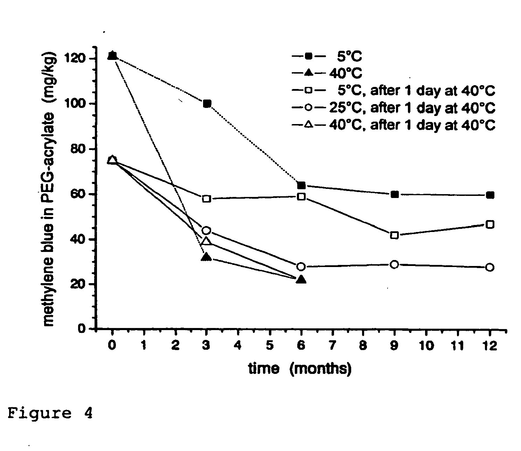

The content of methylene blue in 8-arm PEG-acrylate 2k was monitored in time under different simulated storage conditions for samples that were taken from the freezer and for samples that had been prestressed at 40°C for 1 day (figure 4). For all conditions the methylene blue content decreased in time, indicating that methylene blue was consumed while exerting its stabilizing function. After 6 months, methylene blue concentrations reached approximately constant values, which were higher for lower temperatures. The prestressed samples showed a clearly lowered methylene blue content at the start of the measurements, but levels of prestressed and non-prestressed samples became similar after some time at 40°C.

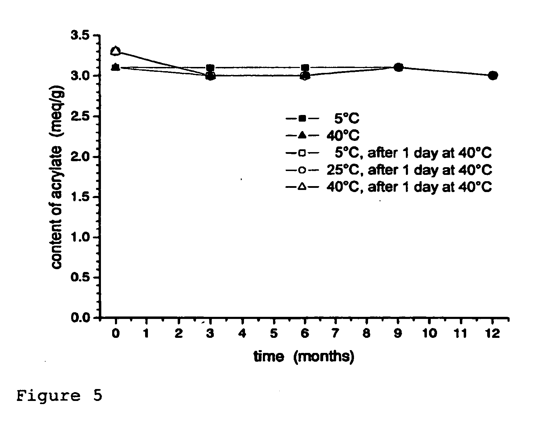

After 6 months at 40°C, some of the 8-arm PEG-acrylate 2k samples had partly or completely polymerized, indicating that a lower limit exists, under which the stabilizing effect of methylene blue is not given anymore. Figure 5 shows that the content of acrylate groups in the 8-arm PEG-acrylate 2k samples that had not polymerized remained constant over the whole observation period, which is another proof that the methylene blue present in the samples protects the acrylate groups from reacting. As becomes clear from figure 4, the effect of methylene blue is enhanced by choosing appropriately low storage temperatures.

### Claims

1. A composition comprising a polymerizable compound with at least one conjugated unsaturated group and methylene blue to prevent premature polymerization of the polymerizable compound, **characterized in that** said composition contains methylene blue in a concentration of 10 to 5000 ppm and that the polymerizable compound comprises a core which carries 2 to 10 chains comprising said conjugated unsaturated groups, whereby said conjugated unsaturated groups are attached to any of the last 20 atoms of the chain.

2. A composition according to claim 1, **characterized in that** said composition contains methylene blue in a concentration of 20 to 1000 ppm.

3. Composition according to any of the preceding claims comprising less than 5%, preferably less than 2% oxygen.

4. Composition according to claim 1 to 3 comprising less than 0.5% oxygen.

5. Composition according to claim 1 to 4, wherein the conjugated unsaturated group of the polymerizable compound is selected from the group consisting of acrylates, acrylamides, quinines, and 2- or 4 vinylpyridiniums.

6. Composition according to claim 1, wherein the chains are poly(ethylene glycol) molecules.

7. Composition according to claim 6, wherein the polymerizable compound is a 4 to 8-arm poly(ethylene glycol) acrylate.

8. Composition according to any of the preceding claims for use in medicine.

9. Use of the composition according to claims 1 to 7 in dentistry.

10. Use of 10 to 5000 ppm methylene blue in a composition comprising a polymerizable compound with at least one conjugated unsaturated group as inhibitor to prevent premature polymerization of the polymerizable compound, wherein the polymerizable compound comprises a core which carries 2 to 10 chains comprising said conjugated unsaturated groups, whereby said conjugated unsaturated groups are attached to any of the last 20 atoms of the chain.

### Patentansprüche

1. Zusammensetzung umfassend eine polymerisierbare Verbindung mit mindestens einer konjugierten ungesättigten Gruppe und Methylenblau, um eine frühzeitige Polymerisation der polymerisierbaren Verbindung zu verhindern, **dadurch gekennzeichnet, dass** diese Zusammensetzung Methylenblau in einer Konzentration von 10 bis 5000 ppm enthält und dass die polymerisierbare Verbindung einen Kern umfasst, welcher 2 bis 10 Ketten trägt, die diese konjugierten ungesättigten Gruppen umfassen, wobei diese konjugierten ungesättigten Gruppen an irgendeinem der letzten 20 Atome der Kette befestigt sind.

**2.** Zusammensetzung gemäss Anspruch 1, **dadurch gekennzeichnet, dass** diese Zusammensetzung Methylenblau in einer Konzentration von 20 bis 1000 ppm enthält.

**3.** Zusammensetzung gemäss einem der vorhergehenden Ansprüche, umfassend weniger als 5%, vorzugsweise weniger als 2% Sauerstoff.

**4.** Zusammensetzung gemäss einem der Ansprüche 1 bis 3, umfassend weniger als 0.5% Sauerstoff.

**5.** Zusammensetzung gemäss einem der Ansprüche 1 bis 4, wobei die konjugierte ungesättigte Gruppe der polymerisierbaren Verbindung ausgewählt ist aus der Gruppe bestehend aus Acrylaten, Acrylamiden, Chininen und 2- oder 4-Vinylpyridinia.

**6.** Zusammensetzung gemäss Anspruch 1, wobei die Ketten Polyethylenglycol-Moleküle sind.

**7.** Zusammensetzung gemäss Anspruch 6, wobei die polymerisierbare Verbindung ein 4- bis 8-Arm-Polyethylenglycolacrylat ist.

**8.** Zusammensetzung gemäss einem der vorhergehenden Ansprüche zur Verwendung in der Medizin.

**9.** Verwendung der Zusammensetzung gemäss einem der Ansprüche 1 bis 7 in der Zahnmedizin.

**10.** Verwendung von 10 bis 5000 ppm Methylenblau in einer Zusammensetzung umfassend eine polymerisierbare Verbindung mit mindestens einer konjugierten ungesättigten Gruppe als Inhibitor, um eine frühzeitige Polymerisation der polymerisierbaren Verbindung zu verhindern, wobei die polymerisierbare Verbindung einen Kern umfasst, welcher 2 bis 10 Ketten trägt, die diese konjugierten ungesättigten Gruppen umfassen, wobei diese konjugierten ungesättigten Gruppen an irgendeinem der letzten 20 Atome der Kette befestigt sind.

## Revendications

**1.** Composition comprenant un composé polymérisable renfermant au moins un groupe insaturé conjugué et du bleu de méthylène pour empêcher la polymérisation prématurée du composé polymérisable, **caractérisée en ce que** ladite composition contient du bleu de méthylène en une concentration de 10 à 5000 ppm et **en ce que** le composé polymérisable comprend un coeur qui porte de 2 à 10 chaînes renfermant lesdits groupes insaturés conjugués, lesdits groupes insaturés conjugués étant fixés à aucun atome parmi les 20 derniers atomes de la chaîne.

**2.** Composition selon la revendication 1, **caractérisée en ce que** ladite composition contient du bleu de méthylène en une concentration de 20 à 1000 ppm.

**3.** Composition selon l'une quelconque des revendications précédentes, comprenant moins de 5%, de préférence moins de 2% d'oxygène.

**4.** Composition selon les revendications 1 à 3, comprenant moins de 0.5% d'oxygène.

**5.** Composition selon les revendications 1 à 4, dans laquelle le groupe insaturé conjugué du composé polymérisable est choisi parmi le groupe constitué d'acrylates, d'acrylamides, de quinines et de 2-ou 4-vinylpyridiniums.

**6.** Composition selon la revendication 1, dans laquelle les chaînes sont des molécules de polyéthylèneglycol.

**7.** Composition selon la revendication 6, dans laquelle le composé polymérisable est un acrylate de polyéthylèneglycol à 4-8 bras.

**8.** Composition selon l'une quelconque des revendications précédentes pour une utilisation en médecine.

**9.** Utilisation de la composition selon l'une quelconque des revendications 1 à 7 en dentisterie.

**10.** Utilisation de 10 à 5000 ppm de bleu de méthylène dans une composition comprenant un composé polymérisable renfermant au moins un groupe insaturé conjugué en tant qu'inhibiteur pour empêcher la polymérisation prématurée du composé polymérisable, où le composé polymérisable comprend un coeur qui porte de 2 à 10 chaînes renfermant lesdits groupes insaturés conjugués, lesdits groupes insaturés conjugués étant fixés à aucun atome parmi les 20 derniers atomes de la chaîne.

Figure 1

Figure 2

8-arm PEG-acrylate 2k (batch# CM030703) at 70°C under Ar

Figure 3

Figure 4

Figure 5

**EP 1 764 404 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0192584 A
- WO 0044808 A
- US 4012310 A
- GB 942318 A
- DD 215699 A

**Non-patent literature cited in the description**

- **S. Schulze ; H. Vogel.** *Chem. Eng. Technol.,* 1998, vol. 21, 829-836
- **Levy.** *J. Polymer Science: Part A: Polymer Chemistry,* 1992, vol. 30, 569-576
- **Mitchell, S.C.** the toxicity of phenothiazine. *Drug Metabolism and Drug Interactions,* 1994, vol. 11, 204-235